# EUROPÄISCHE PATENTSCHRIFT

(11) **EP 1 284 254 B1**
(45) Veröffentlichungstag und Bekanntmachung des Hinweises auf die Patenterteilung: **31.05.2006**
(21) Anmeldenummer: 01119451.1
(22) Anmeldetag: 13.08.2001
(51) Int. Cl.: C07C 51/41, G03C 5/26

(54) **Verfahren zur Herstellung von Dicarbonsäuresalzen für fotografische Zwecke**
Process for the preparation of dicarbonic acid salts for photographic purposes
Procédé pour la préparation de sels d'acides dicarboniques pour usage photographique

(43) Veröffentlichungstag der Anmeldung: 19.02.2003
(73) Patentinhaber: TETENAL AG & Co. KG., 22844 Norderstedt (DE)
(72) Erfinder: Müller, Burkhardt, Dr., 23845 Itzstedt (DE); Hengefeld, Axel, Dr., 20249 Hamburg (DE); Steinebach, Frank, 22529 Hamburg (DE)
(74) Vertreter: Glawe, Delfs, Moll

(56) Entgegenhaltungen:
- EP-A- 0 710 621
- EP-A- 0 913 190

## Beschreibung

Die Erfindung betrifft ein Verfahren zum Herstellen eines Ammonium salzes einer Di- oder Tricarbonsäure sowie die Verwendung des so hergestellten Salzes zur Herstellung von als Feststoff formulierten fotografischen Chemikalien, insbesondere Bleich- oder Bleichfixierbädern

EP-A-0 710 621 offenbart die Produktion von Kohlendioxid zur Konservierung von Obst und Gemüse oder der Wachstumsstimulation von Pflanzen durch Reaktion von bestimmten Säuren mit einem Alkalicarbonat, beispielsweise einem Hydrogencarbonat, bei dem im Ergebnis ein Alkalisalz einer Di-oder Tricarbonsäure entstehen kann.

Belichtete Silberhalogenidfilme werden in kommerziellen Labors vollautomatisch entwickelt und verarbeitet. Farbnegativfilme beispielsweise durchlaufen dabei ein Entwicklerbad, ein Bleichbad, ein Fixierbad und in der Regel ein Stabilisierbad bzw. mehrere Waschschritte. Die Schritte des Bleichens und Fixierens können ggf. mittels eines Bleichfixierbades in einem einzigen Verarbeitungsschritt kombiniert werden. Die fertig entwickelten Negative werden auf Colorpapier belichtet, das belichtete Colorpapier durchläuft anschließend ebenfalls ein Entwicklungsbad, in der Regel ein kombiniertes Bleichfixierbad und abschließende Waschschritte. Die Waschschritte können ggf. durch ein Stabilisierbad ersetzt werden.

Die verschiedenen Verarbeitungsbäder erschöpfen sich im Laufe der Verwendung. Während ein Fotoamateur erschöpfte Bäder in der Regel auswechselt, erfolgt bei kommerziellen Fotolabors eine Auffrischung verbrauchter Lösungen. Im Handel sind zu diesem Zweck die entsprechenden Prozeßchemikalien als Flüssigkonzentrate oder Pulver erhältlich. Flüssigkonzentrate benötigen aufgrund ihres Wasseranteils bei Transport und Lagerung verhältnismäßig viel Platz. Die leeren Konzentratbehälter stellen ein erhebliches Abfallvolumen dar.

Chemikalien in Pulverform sind schwierig zu handhaben. Da die Pulver bei der Handhabung stauben können, müssen Vorkehrungen zur Vermeidung gesundheitlicher Beeinträchtigungen der Anwender getroffen werden.

Es ist daher bereits vorgeschlagen worden (EP-A 0 678 782), fotografische Prozeßchemikalien als Granulat zu formulieren. Es werden verschiedene Varianten der Feuchtgranulierung (beispielsweise Wirbelschichtgranulierung) eingesetzt.

Bleichbäder haben im allgemeinen die Aufgabe, metallisches Silber in der Emulsion zu oxidieren, das anschließend im Fixierschritt aus der Emulsion komplexometrisch herausgelöst wird. Bei Farbnegativfilmen (C-41 Prozeß) schließt sich das Bleichbad unmittelbar an den Entwicklungsprozeß an. Daher muss ein Bleichbad, neben einem entsprechenden (geeigneten) Redoxpotential, auch eine ausreichende Pufferung besitzen, um den pH-Wert des Bleichbades nach Einschleppung des alkalischen Entwicklers konstant zu halten. Außerdem muß eine schnelle pH-Wert Senkung innerhalb der Filmemulsion erfolgen, damit keine unerwünschte Entwickleroxidations-Produkt(EOP)-Kupplung erfolgt und kein Bleichschleier (Bleach Induced Fog-BIF) resultiert. Auch muß ein geeigneter pH-Wert des Bleichbades realisiert sein, damit das Oxidationsmittel, in der Regel ein Fe(III)-Komplex, ein ausreichendes Oxidationspotential zur Oxidation des Silbers besitzt. In der Regel liegen Bleichbäder in einem pH-Wert Bereich zwischen 4 und 6.

Es ist bekannt, daß organische Säuren (z.B. Essigsäure pKs=4,75) mit einem pKs-Wert in dem gewünschten pH-Bereich von 4-6 die Eigenschaften der Pufferung und der Bleichschleier-Vermeidung erfüllen. Bei einer festen Formulierung eines Bleichbades können flüssige Säuren oder hygroskopische und flüchtige Salze (wie Ammoniumacetat) nicht verwendet werden. Eine geeignete feste Säure mit einem pKS-Wert in dem gewünschten Bereich ist Bernsteinsäure (pKsI=4,21). Organische Dicarbonsäuren mit dem entsprechenden pKS-Wert und deren Salze haben die Eigenschaften, im pH-Bereich des Bleichbades ausreichend zu puffern, keinen BIF zu bilden und wegen des niedrigen Dampfdruckes im Gegensatz zur flüssigen, stark riechenden Essigsäure keine Geruchsbelästigung darzustellen. Es wurde gefunden, daß eine Kombination aus dem Diammoniumsalz der Bernsteinsäure (Ammoniumsuccinat) und Maleinsäure ein optimales Säure/Salzpaar darstellt, welches die Bedingungen Geruchlosigkeit, pKS-Wert im richtigen Bereich und gute Pufferung erfüllt und in Bleichbadformulierungen zu sehr guten Bleichergebnissen führt. Ebenfalls eingesetzt werden können Ammonium salze sonstiger Di- oder Tricarbonsäuren.

Diese Di- oder Tricarbonsäuresalze sind kommerziell nicht oder nur zu sehr hohen Preisen zu erwerben. Der Erfindung liegt daher die Aufgabe zugrunde, ein Verfahren zur einfachen und kostengünstigen Herstellung der eingangs genannten Salze zu schaffen, bei dem man das Reaktionsprodukt in einer Form erhält, die ohne aufwendige Nachbehandlung die Weiterverarbeitung zu als Feststoff formulierten fotografischen Chemikalien ermöglicht.

Die Erfindung löst diese Aufgabe dadurch, daß man die gewünschten Di- und/oder Tricarbonsäuren einerseits (ggf. in Form der Säureanhydride) sowie ein Ammonium hydrogencarbonat andererseits reagieren läßt in einer Mischung, die einen Anteil Reaktionsvermittler von 15 Gew.-% oder weniger enthält.

Der Begriff Reaktionsvermittler bezeichnet im Rahmen der Erfindung jegliche Stoffe oder Stoffgemische, die die gewünschte Säure-Basen-Reaktion zwischen den als Feststoffen zugegebenen Edukten fördern und gewissermaßen als Reaktionsmedium dienen. Es können sich bspw. um eine geringe Menge Wasser handeln, die aus den zugesetzten Edukten eine hochviskose Mischung entstehen läßt und so die gewünschte Reaktion fördert. Wasser als Reaktionsvermittler muß nicht unbedingt vor Beginn der Reaktion zugegeben werden, es kann auch in ausreichender Menge durch die Zersetzung des Ammoniumhydrogencarbonats bzw. die stattfindende Säure-Basen-Reaktion in situ entstehen und anschließend als in der Reaktionsmischung verbleibendes Wasser eine weitgehend vollständige Säure-Basen-Reaktion zwischen den Reaktionspartnern fördern.

Der Zusatz eines polymeren Reaktionsvermittlers kann sinnvoll sein. Es handelt sich vorzugsweise um einen bezüglich der gewünschten Säure-Basen-Reaktion inerten Hilfsstoff, der bei der Reaktionstemperatur (vorzugsweise 40 bis 100°C) soweit erweicht, daß er gegenüber den als Feststoffen zugegebenen Edukten eine Binde- bzw. Granulierwirkung übernehmen kann. Für diesen Zweck geeignete Polymere sind detailliert in EP-A-0 913 190 beschrieben. Im Rahmen der Erfindung sind besonders bevorzugt Polyethylenglykole (Molekulargewicht bevorzugt zwischen 4.000 und 10.000) sowie s-Caprolactam. Der Anteil dieser Polymere an der Reaktionsmischung liegt bevorzugt zwischen 2 und 10 Gew.-%, weiter vorzugsweise zwischen 3 und 6 Gew.-%.

Wenn im Rahmen der Erfindung der Reaktionsvermittler einen bestimmten Anteil an der Reaktionsmischung nicht überschreiten soll, bedeutet dies, daß das Gewicht ursprünglich zugesetzten und/oder im Verlaufe der Reaktion entstehenden Reaktionsvermittlers (wie bspw. Wasser) einen bestimmten Gewichtsanteil (in Anspruch 1 15%) am Gewicht der ursprünglich eingesetzten Reaktionsmischung nicht überschreiten soll. Diese Bezugnahme auf die ursprünglich eingesetzte Reaktionsmischung ist wichtig, da die Gesamtmasse der Reaktionsmischung sich im Verlaufe der Reaktion durch Entweichen von Kohlendioxid ändert.

Der Kern der Erfindung liegt darin, die gewünschten Carbonsäuresalze durch eine Reaktion zwischen festen Edukten herzustellen und dabei Lösemittel (Wasser) bzw. Bindemittel nur in einem so geringen Umfang zuzusetzen, daß die gesamte Reaktion in weitgehend fester bzw. hochviskoser Phase stattfindet. Dennoch erhält man eine weitgehend stöchiometrische Umsetzung zum gewünschten Salz, das als Reaktionsprodukt sofort in Form des gewünschten Feststoffes vorliegt und ohne aufwendige Nachbehandlung zu fotografischen Reagenzien wie bspw. als Feststoff formulierten Bleichbädern weiterverarbeitet werden kann.

Ein besonderer Vorteil der Erfindung liegt darin, daß das Verfahren in sogenannten Schmelz- oder Granuliermischern durchgeführt werden kann, die man zur Herstellung von als Feststoff formulierten fotografischen Reagenzien sowieso benötigt. Das erfindungsgemäße Verfahren kann somit bei Herstellern von Fotochemikalien ohne besonderen Aufwand mit der vorhandenen technischen Ausrüstung durchgeführt werden. Insbesondere kann die Reaktion in sogenannten Pflugscharmischern durchgeführt werden, die eine gute Durchmischung fester bzw. hochviskoser Stoffgemische ermöglichen.

Die Reaktionsgeschwindigkeit kann durch Wärmezufuhr erhöht werden, dies kann vorzugsweise mittels eines Heizmantels (in der Regel ein beheizbarer Doppelmantel) des Mischbehälters erfolgen.

Als Edukte für das erfindungsgemäße Verfahren besonders geeignete Di-/Tricarbonsäuren sind Oxalsäure, Malonsäure, Bernsteinsäure, Glutarsäure, Adipinsäure, Maleinsäure, Weinsäure, Citronensäure und Äpfelsäure. Als Dicarbonsäureanhydride können insbesondere Maleinsäureanhydrid, Bernsteinsäureanhydrid und Glutarsäureanhydrid eingesetzt werden.

Ein Weiteres Edukt der Reaktion ist Ammoniumhydrogencarbonat.

Ammoniumhydrogencarbonat zerfällt beim Erwärmen ab etwa 60°C in Ammoniak, Wasser und Kohlendioxid. Das Ammoniak reagiert mit den Dicarbonsäuren zu den entsprechenden Ammoniumsalzen. Das frei werdende Wasser fungiert als Reaktionsvermittler, so daß sich die Reaktionsgeschwindigkeit beschleunigt und eine quantitative Umsetzung erfolgt. Ammoniakgeruch ist zu Beginn der Reaktion kaum wahrnehmbar und tritt erst bei vollständiger Umsetzung in geringem Umfang auf. Somit kann ein leichter Ammoniakgeruch als Indikator für die Beendigung der Reaktion benutzt werden. Bei ständiger Durchmischung der als Feststoff zugesetzten Edukte beträgt die Dauer der Reaktion abhängig von der Größe der Charge und der zugeführten Wärme etwa 15 min. Während der Reaktion entweichen fortlaufend Kohlendioxid und Wasserdampf.

Vorzugsweise werden die Edukte Säure und Hydrogencarbonat im stöchiometrischem Verhältnis zugesetzt. Die Reaktion verläuft weitgehend quantitativ, da durch das entweichende Kohlendioxid das Gleichgewicht vollständig auf diese Seite des Carbonsäuresalzes verschoben wird.

Das Reaktionsprodukt des erfindungsgemäßen Verfahrens kann unmittelbar zu fotografischen Reagenzien weiterverarbeitet werden. Sofern die Restfeuchte für den vorgesehenen Verwendungszweck zu hoch ist, kann eine Trocknung erfolgen, die vorzugsweise unmittelbar im Reaktionsgefäß (dem Mischbehälter) stattfinden kann und durch verminderten Druck und/oder Temperaturen von 40 bis 100°C, vorzugsweise etwa 60 bis 80°C, gefördert werden kann.

Gegenstand der Erfindung ist ferner die Verwendung eines so hergestellten Salzes zur Herstellung von als Feststoff formulierten fotografischen Reagenzien. Diese können insbesondere als Granulat oder als Formkörper (Tablette oder dergleichen) formuliert sein. Besonders vorteilhaft ist die vorliegende Erfindung zur Herstellung von Bleich- oder Bleichfixierbädern. Die Formulierung der fotografischen Reagenzien kann insbesondere durch Schmelzgranulation erfolgen, wie sie in EP-A-0 913 190 beschrieben ist.

### Beispiel 1

Herstellung von Ammonium salzen aus Dicarbonsäuren und Anhydriden im Labormaßstab

Alle Versuche wurden in einem Lödige® M5 Horizontal-Pflugscharmischer mit Messerkopf und beheizbarem Doppelmantel mit 5 l Fassungsvermögen durchgeführt. Die Umsetzungen wurden bei einer Manteltemperatur von 80°C durchgeführt.

Das Verhältnis Säuren/Anhydride zu den entsprechenden Hydrogencarbonaten wurde so gewählt, daß ein äquimolarer Umsatz stattfinden kann (Molverhältnis 1 : 2). Die Reaktanden (Edukte) wurden in die Granulationskammer gegeben, Pflugscharen und Messerkopf angestellt und der Produkttemperaturanstieg verfolgt. Im Abstand von 5 min wurde in der Reaktionskammer die Entwicklung von Kohlendioxid kontrolliert. Kohlendioxidfreisetzung ist in der Reaktionsmischung an einem Knistern zu hören. Als zusätzliche Detektion des Endpunktes wurde ein schwacher Ammoniakgeruch wahrgenommen. Nach Beendigung der Reaktion (Reaktionsdauer 10 - 20 min) wurde das Produkt aus dem Granulator entfernt. Tabelle 1 zeigt eine Aufstellung der 12 durchgeführten Salzreaktionen:

**Tabelle 1 Kombinationen der Salzherstellung im Lödige M5**

| | [kg] | NH₄HCO₃ [kg] |
|---|---|---|
| Oxalsäure | 1,25 | 2,20 |
| Malonsäure | 1,25 | 1,90 |
| Bernsteinsäure | 1,25 | 1,67 |
| Glutarsäure | 1,25 | 1,50 |
| Adipinsäure | 1,25 | 1,35 |
| Maleinsäure | 1,25 | 1,70 |
| Weinsäure | 1,25 | 1,32 |
| Citronensäure | 1,25 | 1,03 |
| Äpfelsäure | 1,25 | 1,47 |
| Maleinsäureanhydrid | 1,25 | 2,02 |
| Bernsteinsäureanhydrid | 1,25 | 1,98 |
| Glutarsäureanhydrid | 1,25 | 1,73 |

Die erhaltenen Salze wurden in einem Trockenschrank bei 65°C getrocknet. Für eine erste Prüfung auf vollständige Umsetzung wurden die Salze in Wasser gelöst. Bei unvollständiger Salzbildung wäre bei diesem Lösevorgang eine Kohlendioxidbildung aufgrund von Restmengen von Hydrogencarbonat zu erwarten. Diese trat nicht auf.

Anschließend wurden die erhaltenen Salze in einer Konzentration von 0,5 mol/l in Wasser gelöst und der pH-Wert der Lösung gemessen. Als Referenz wurden Lösungen gleicher Konzentration der gleichen Salze angesetzt, die auf herkömmliche Weise in flüssiger Phase in einem Lösungsmittel hergestellt wurden. Die Ergebnisse sind in Tabelle 2 einander gegenüber gestellt. Von den 12 Kombinationen der Tabelle 1 sind in Tabelle 2 nur 9 Kombinationen aufgeführt, da sich für aus Säureanhydriden hergestellte Salze identische ph-Werte ergeben wie bei der Herstellung aus den entsprechenden Dicarbonsäuren. Die pH-Werte stimmen im Rahmen der Meßgenauigkeit überein, was ein weiteres Indiz für einen vollständigen Reaktionsablauf bei dem erfindungsgemäßen Verfahren ist.

**Tabelle 2**

| | pH-Wert 0,5 mol/l Flüssigansatz | pH-Wert 0,5 mol/l Salz aus Granulator |
|---|---|---|
| Ammoniumoxalat | 7,26 | 7,18 |
| Ammoniummalonat | 6,31 | 6,42 |
| Ammoniumsuccinat | 6,28 | 6,37 |
| Ammoniumglutarat | 6,20 | 6,17 |
| Ammoniumadipinat | 6,30 | 6,21 |
| Ammoniumtartrat | 6,12 | 6,25 |
| Ammoniumcitrat | 5,06 | 5,07 |
| Ammoniummalat | 6,03 | 6,12 |
| Ammoniummaleinat | 6,42 | 6,43 |

In den nachfolgenden Beispielen 2 und 3 wird die Herstellung von Ammoniumsuccinat im Technikumsmaßstab beschrieben.

### Beispiel 2

### Lödige® Pflugscharmischer FM 130

Zur Verwendung der Methode im Technikums-/Produktionsmaßstab wurde Ammoniumsuccinat im Lödige® Pflugscharmischer FM 130 hergestellt. Hierbei wurden 34,55 kg Bernsteinsäure und 46,51 kg Ammoniumhydrogencarbonat (molares Verhältnis 1: 2) in den Mischer gegeben und die Manteltemperatur auf 80°C eingestellt. Die Pflugscharen wurden auf eine Geschwindigkeit von 160 U/min eingestellt und der Messerkopf auf low speed gestellt. Es wurden in Abhängigkeit von der Zeit die Produkttemperatur und die Stromstärke-Aufnahme der Pflugscharen gemessen. Mit beiden Kenngrößen kann die Reaktion verfolgt werden, sie sind in Tabelle 3 dargestellt.

**Tabelle 3 Daten zur Reaktionskontrolle bei der Herstellung von Ammoniumsuccinat im Lödige FM 130**

| Zeit [min] | Mantel- temperatur [°C] | Produkt- temperatur [°C] | Pflugscharen-Stromaufnahme [A] |
|---|---|---|---|
| 4 | 80 | 16 | 9 |
| 10 | 80 | 18 | 9 |
| 20 | 80 | 20 | 11 |
| 30 | 80 | 20 | 11 |
| 40 | 80 | 23 | 11 |
| 50 | 80 | 27 | 11,2 |
| 60 | 80 | 39 | 10,5 |
| 70 | 80 | 50 | 10 |

Die Werte in Tabelle 3 verdeutlichen die visuell beobachteten Phänomene. Zu Beginn der Reaktion stieg die Manteltemperatur auf die gewünschten 80°C an, jedoch blieb das Produkt eine halbe Stunde lang bei einer Temperatur von ca. 20°C konstant. Zusätzlich stieg die Stromabnahme der Pflugscharen an, da in diesem Schritt der Reaktion Wasser freigesetzt wird und die Viskosität der Mischung zunimmt. Nach ca. 40 min ist ein stärkerer kontinuierlicher Temperaturanstieg zu verzeichnen. Die Reaktion vervollständigt sich, die Wasserfreisetzung erreicht ihr Maximum und die Viskosität der Reaktionsmischung nimmt wieder ab. Daher sinkt die Stromaufnahme der Pflugscharen. Der Wärmeeintrag in die Mischung wird nicht mehr durch die endotherme Reaktion verbraucht und die Produkttemperatur steigt bis auf 50°C an. Die Entleerung des Rohproduktes erfolgte bei 50°C. Die Konsistenz des Salzes war bei diesen Temperaturen cremig-fest und ging bei Erkalten in einen amorphen, spröderen Zustand über. Anschließend wurde das Produkt unter Vakuum getrocknet. Es wurden 45 kg Ammoniumsuccinat erhalten. Während dieser Reaktion wurden theoretisch 10,6 l Wasser und 13,2 m³ Kohlendioxid freigesetzt.

Das so produzierte und getrocknete Ammoniumsuccinat wurde in einem durch Schmelzgranulation hergestelltem C-41 Bleichbad-Granulat verwendet. Die Herstellung des Granulates wird in Beispiel 4 beschrieben.

### Beispiel 3

### Hochleistungs-Knetmaschine IKA HKV 50 VHV

Zur Verwendung der Methode im Technikums-/Produktionsmaßstab wurde Ammoniumsuccinat im IKA Knetmischer HKV 50 VHV hergestellt. Hierbei wurden 10,55 kg Bernsteinsäure und 14,13 kg Ammoniumhydrogencarbonat (molares Verhältnis 1 : 2) in den Kneter gegeben und die Manteltemperatur auf 80°C eingestellt. Es wurde in Abhängigkeit von der Zeit die Produkttemperatur im Reaktionsgefäß verfolgt. Die aufgenommenen Parameter sind in Tabelle 4 dargestellt.

**Tabelle 4 Daten zur Reaktionskontrolle bei der Herstellung von Ammoniumsuccinat im IKA Knetmischer HKV 50 VHV**

| | | |
|---|---|---|
| Zeit [min] | Manteltemperatur [°C] | Produkttemperatur [°C] |
| 4 | 80 | 16 |
| 10 | 80 | 18 |
| 20 | 80 | 20 |
| 30 | 80 | 20 |
| 40 | 80 | 24 |
| 50 | 80 | 28 |
| 60 | 80 | 41 |
| 70 | 80 | 51 |

Nach Abschluß der Reaktion wurde das Reaktionsgefäß auf einen Absolutdruck von ca. 20 mbar evakuiert und das Produkt bei einer Manteltemperatur von 60°C über 16 h getrocknet. Erhalten wurden 13,6 kg eines weißen feinkristallinen Produktes.

Das so produzierte und getrocknete Ammoniumsuccinat wurde in einem durch Schmelzgranulation hergestelltem C-41 Bleichbad-Granulat verwendet. Die Herstellung des Granulates wird in Beispiel 4 beschrieben.

### Beispiel 4

### Herstellung eines C-41 Bleichbad-Granulates mittels Schmelzgranulation

Das Prinzip der Schmelzgranulation ist die Herstellung einer staubfreien Feststoffmischung mit einem niedrigschmelzenden Binder. Der Schmelzbereich des Binders kann zwischen 40-140°C liegen. Entscheidend ist, daß die zu granulierenden Rohstoffe, im Vergleich zum Binder, sämtlich einen höheren Schmelzpunkt besitzen. Des weiteren dürfen die zu granulierenden Rohstoffe bei den erhöhten Granulationstemperaturen keine chemische Reaktion untereinander oder mit dem Binder eingehen.

Durch die gezielte Variation der Bindermengen kann der Grad der Agglomeration beeinflusst werden. Die Bildung von Granulatpartikeln und die Korngrößenverteilung eines Granulates hängt von der Viskostät des geschmolzenen Binders, der eingesetzten Menge an Binder, der Granulationsdauer und der Drehzahl der Pflugscharen ab. Bei Kenntnis dieser Parameter kann das Partikelgrössenspektrum eines Granulates gesteuert werden.

Für die Herstellung eines C-41 Bleichregenerator-Granulates wurden die in Tabelle 5 angegebenen Substanzen für 1 l Regenerator (gebrauchsfertige flüssige Lösung zur Regenerierung eines Bleichbades) verwendet:

Ammoniumeisenpropylendiamintetraessigsäure (NH₄Fe-PDTA), Ammoniumbromid (NH₄Br), Ammoniumsuccinat und Maleinsäure. Als Binder wurde Polethylenglykol 10000 (PEG 10000) verwendet.:

**Tabelle 5 feste Formulierung eines C-41 Bleichbades**

| Substanz | [g] |
|---|---|
| NH₄Fe-PDTA | 107,10 |
| NH₄Br | 82,40 |
| Ammoniumsuccinat | 50,00 |
| Maleinsäure | 58,18 |
| PEG 10000-Binder | 28,74 |

Diese Rezeptur ergibt sehr gute Bleichergebnisse, hat eine ausreichende Pufferkapazität und verhindert Bleichschleier. Bei der Granulation dieser Rezeptur werden die in Tabelle 5 aufgeführten Substanzen mit Ausnahme von Maleinsäure in einem Granulator mit Pflugscharen und beheizbarem Doppelmantel bei ca. 60°C schmelzgranuliert. Die Bindermenge ist so gewählt, daß ein staubfreies und rieselfähiges Granulat entsteht. Maleinsäure kann nicht granuliert werden, da sie bei erhöhten Temperaturen Reaktionen mit anderen Inhaltsstoffen eingeht. Zum Beispiel reagiert Maleinsäure mit Ammoniumsuccinat bei 60°C zu Ammoniummaleinat und Bernsteinsäure. Bernsteinsäure löst sich bei dem gewünschten pH-Wert von 3,90 nicht ausreichend in der Regenerator-Lösung, so daß die erforderliche Pufferkapazität nicht erreicht wird und der Bleichbad-Regenerator somit unbrauchbar ist. Aus diesem Grund wird Maleinsäure dem final blend zugegeben und mit dem Granulat zu einer homogenen Mischung vereinigt. Dadurch ist es möglich, den pH-Wert des Bleichbad-Regenerators nach jedem Granulationsbatch neu einzustellen, da in den Rohstoffen eine gewisse pH-Wertschwankung auftritt. Zur Durchführung im Technikums-/Produktionsmaßstab wurden 23,96 kg NH₄Fe-PDTA, 18,43 kg NH₄Br, 11,18 kg Ammoniumsuccinat mit 6,43 kg PEG 10000 als Binder im Lödige® Pflugscharmischer FM 130 granuliert. Die Manteltemperatur wurde auf 75°C eingestellt. Die Pflugscharen wurden auf eine Geschwindigkeit von 160 U/min und die Messerkopfgeschwindigkeit auf high speed eingestellt. Es wurden in Abhängigkeit von der Zeit die Produkttemperatur und die Stromstärke-Aufnahme der Pflugscharen gemessen. Mit diesen Kenngrößen wurde die Reaktion verfolgt (Tabelle 6).

**Tabelle 6 Daten zur Reaktionskontrolle bei der Herstellung des C-41 Bleichbadgranulates im Lödige FM 130**

| Zeit [min] | Produkt- Temperatur [°C] | Pflugscharen-Stromaufnahme [A] |
|---|---|---|
| 0:00 | 18 | 7,4 |
| 10:00 | 44 | 7,2 |
| 20:00 | 57 | 7,2 |
| 30:00 | 58 | 7,1 |
| 40:00 | 62 | 7,2 |
| 45:00 | 63 | 7,6 |

Die Werte in Tabelle 6 veranschaulichen den Verlauf des Granulationsprozesses. In den ersten 20 min erfolgt ein Wärmeeintrag in die Pulvermischung. Dabei steigt die Temperatur konstant an. Auch die Viskosität der Mischung, die über die Stromaufnahme der Pflugscharen verfolgt werden kann, verändert sich nicht. Nach 20 min beim Schmelzpunkt des PEG 10000 (ca. 58°C) bleibt die Temperatur der Feststoffmischung weitestgehend konstant. Sämtlicher Wärmeeintrag wird zum Schmelzen des Binders verwendet. Der Granulationsvorgang beginnt mit dem Schmelzen des Binders. Dieser Vorgang ist auch über den Anstieg der Stromstärke-Aufnahme der Pflugscharen zu beobachten. Nach 45 min Prozeßzeit und einer visuellen Kontrolle der Granulatmischung wurde das fertige Produkt aus dem Mischer entfernt und abgekühlt. In einem Containermischer wurden 13,3 kg Maleinsäure mit 60,0 kg des erhaltenen Schmelzgranulates gemischt. Das erhaltene C-41 Bleichbad-Granulat hatte folgende Korngrößenverteilung:

| Siebe: | µm <250 | µm >250 | µm >355 | µm >500 | µm >710 | mm >1,0 | mm >1,4 | mm >2,0 | mm >2,8 |
|---|---|---|---|---|---|---|---|---|---|
| Anteil Siebgut [%] | 0,29 | 0,70 | 3,11 | 10,24 | 17,57 | 21,90 | 22,80 | 14,81 | 8,54 |

Das Maximum der Korngrößenverteilung liegt mit ca. 40% zwischen 1,0 und 2,0 mm Korngröße. Das erhaltene Granulat ist staubfrei und gut rieselfähig. Lagerungstests bei 35°C und 50°C über 4 Wochen zeigten keine wesentlichen Veränderungen der Granulatstruktur und Rieselfähigkeit. Nach Auflösen von gelagerten Granulaten wurde der richtige pH-Wert und volle uneingeschränkte Bleichleistung erreicht. Das Verfahren ist im Vergleich zu anderen Granulationstechniken (z.B. Wirbelschichtgranulation) wesentlich zeitsparender und kostengünstiger.

## Patentansprüche

1. Verfahren zum Herstellen eines Ammoniumsalzes einer Di-oder Tricarbonsäure, **gekennzeichnet durch** Reagierenlassen von Di/Tricarbonsäure und/oder Di/Tricarbonsäureanhydrid einerseits und einem Ammoniumhydrogencarbonat andererseits in einer Mischung, die einen Anteil Reaktionsvermittler von 15 Gew.-% oder weniger enthält.

2. Verfahren nach Anspruch 1, **dadurch gekennzeichnet, daß** der Reaktionsvermittler ausgewählt ist aus der Gruppe bestehend aus Wasser und zwischen 40°C und 100°C erweichbaren Polymeren.

3. Verfahren nach Anspruch 2, **dadurch gekennzeichnet, daß** der Reaktionsvermittler ein zwischen 40°C und 100°C erweichbares Polymer ist und daß sein Anteil an der Reaktionsmischung zwischen 2 und 10 Gew.-%, vorzugsweise 3 und 6 Gew.-%, liegt.

4. Verfahren nach Anspruch 3, **dadurch gekennzeichnet, daß** das Polymer ausgewählt ist aus der Gruppe bestehend aus Polyethylenglykolen und ε-Caprolactam.

5. Verfahren nach Anspruch 2, **dadurch gekennzeichnet, daß** als Reaktionsvermittler in situ entstehendes Reaktionswasser verwendet wird.

6. Verfahren nach einem der Ansprüche 1 bis 5, **dadurch gekennzeichnet, daß** es in einem Mischbehälter durchgeführt wird.

7. Verfahren nach Anspruch 6, **dadurch gekennzeichnet, daß** der Mischbehälter ein Pflugscharmischer ist.

8. Verfahren nach einem der Ansprüche 1 bis 7, **dadurch gekennzeichnet, daß** während der Reaktion Wärme zugeführt wird.

9. Verfahren nach Anspruch 8, **dadurch gekennzeichnet, daß** die Wärmezufuhr mittels eines Heizmantels des Mischbehälters erfolgt.

10. Verfahren nach einem der Ansprüche 1 bis 9, **dadurch gekennzeichnet, daß** die Di/Tricarbonsäure ausgewählt ist aus der Gruppe bestehend aus Oxalsäure, Malonsäure, Bernsteinsäure, Glutarsäure, Adipinsäure, Maleinsäure, Weinsäure, Citronensäure und Äpfelsäure.

11. Verfahren nach einem der Ansprüche 1 bis 9, **dadurch gekennzeichnet, daß** die Dicarbonsäureanhydride ausgewählt sind aus der Gruppe bestehend aus Maleinsäureanhydrid, Bernsteinsäureanhydrid und Glutarsäureanhydrid.

12. Verfahren nach einem der Ansprüche 1 bis 11, **dadurch gekennzeichnet, daß** das Reaktionsprodukt getrocknet wird.

13. Verfahren nach Anspruch 12, **dadurch gekennzeichnet, daß** die Trocknung im Reaktionsgefäß erfolgt.

14. Verfahren nach Anspruch 12 oder 13, **dadurch gekennzeichnet, daß** die Trocknung bei vermindertem Druck und/oder bei Temperaturen von 40 - 100°C, vorzugsweise 60 - 80°C, erfolgt.

15. Verwendung eines nach einem der Ansprüche 1 bis 14 hergestellten Salzes zur Herstellung von als Feststoff formulierten fotografischen Reagenzien.

16. Verwendung nach Anspruch 15, **dadurch gekennzeichnet, daß** das fotografische Reagens ein Bleichbad ist.

17. Verwendung nach Anspruch 15 oder 16, **dadurch gekennzeichnet, daß** die fotografischen Reagenzien durch Schmelzgranulation formuliert werden.

## Claims

1. A method of preparing an ammonium salt of a di or tricarboxylic acid, **characterised by** allowing di/tricarboxylic and/or di/tricarboxylic acid anhydride to react, on the one hand, with an ammonium hydrogen carbonate and, on the other hand, with a mixture which contains a proportion of reaction agent of 15 % by weight or less.

2. A method according to Claim 1, **characterised in that** the reaction agent is selected from the group comprising water and polymers which can be softened between 40°C and 100°C.

3. A method according to Claim 2, **characterised in that** the reaction agent is a polymer which can be softened between 40°C and 100°C, and **in that** its proportion in the reaction mixture is between 2 and 10 % by weight, preferably 3 and 6 % by weight.

4. A method according to Claim 3, **characterised in that** the polymer is selected from the group comprising polyethylene glycols and ε-caprolactam.

5. A method according to Claim 2, **characterised in that** reaction water formed in situ is used as reaction agent.

6. A method according to any one of Claims 1 to 5, **characterised in that** it is carried out in a mixing vessel.

7. A method according to Claim 6, **characterised in that** the mixing vessel is a plough-share mixer.

8. A method according to any one of Claims 1 to 7,
**characterised in that** heat is supplied during the reaction.

9. A method according to Claim 8, **characterised in that** the heat input is provided by means of a heating jacket of the mixing vessel.

10. A method according to any one of Claims 1 to 9, **characterised in that** the di/tricarboxylic acid is selected from the group comprising oxalic acid, malonic acid, succinic acid, glutaric acid, adipic acid, maleic acid, tartaric acid, citric acid and malic acid.

11. A method according to any one of Claims 1 to 9, **characterised in that** the dicarboxylic acid anhydride is selected from the group comprising maleic acid anhydride, succinic acid anhydride and glutaric acid anhydride.

12. A method according to any one of Claims 1 to 11, **characterised in that** the reaction product is dried.

13. A method according to Claim 12, **characterised in that** the drying is carried out in the reaction vessel.

14. A method according to Claim 12 or 13, **characterised in that** the drying is carried out at reduced pressure and/or at temperatures of 40°C to 100°C, preferably 60°C to 80°C.

15. Use of a salt prepared according to any of Claims 1 to 14 for the preparation of photographic reagents formulated as a solid.

16. Use according to Claim 15, **characterised in that** the photographic reagent is a bleaching bath.

17. Use according to Claim 15 or 16, **characterised in that** the photographic reagents are formulated by melt granulation.

## Revendications

1. Procédé de préparation d'un sel d'ammonium d'un acide dicarboxylique ou tricarboxylique, **caractérisé en ce que** l'on fait réagir de l'acide di/tricarboxylique et/ou de l'anhydride d'acide di/tricarboxylique, d'une part, et un hydrogénocarbonate d'ammonium, d'autre part, dans un mélange qui contient une proportion d'agent réactionnel de 15 % en poids ou moins.

2. Procédé selon la revendication 1, **caractérisé en ce que** l'agent réactionnel est choisi dans le groupe constitué de l'eau et des polymères se ramollissant entre 40°C et 100°C.

3. Procédé selon la revendication 2, **caractérisé en ce que** l'agent réactionnel est un polymère se ramollissant entre 40°C et 100°C et **en ce que** sa proportion dans le mélange réactionnel se situe entre 2 et 10 % en poids, de préférence 3 et 6 % en poids.

4. Procédé selon la revendication 3, **caractérisé en ce que** le polymère est choisi dans le groupe constitué des polyéthylène glycols et du ε-caprolactame.

5. Procédé selon la revendication 2, **caractérisé en ce que** l'on utilise comme agent réactionnel une eau réactionnelle engendrée in situ.

6. Procédé selon l'une des revendications 1 à 5, **caractérisé en ce qu'**il est réalisé dans un réservoir de mélange.

7. Procédé selon la revendication 6, **caractérisé en ce que** le réservoir de mélange est un mélangeur à soc de charrue.

8. Procédé selon l'une des revendications 1 à 7, **caractérisé en ce que** de la chaleur est amenée pendant la réaction.

9. Procédé selon la revendication 8, **caractérisé en ce que** l'apport de chaleur se fait au moyen d'une gaine de chauffage du réservoir de mélange.

10. Procédé selon l'une des revendications 1 à 9, **caractérisé en ce que** l'acide di/tricarboxylique est choisi dans le groupe constitué de l'acide oxalique, l'acide malonique, l'acide succinique, l'acide glutarique, l'acide adipique, l'acide maléique, l'acide tartrique, l'acide citrique et l'acide malique.

11. Procédé selon l'une des revendications 1 à 9, **caractérisé en ce que** les anhydrides d'acide dicarboxylique sont choisis dans le groupe constitué de l'anhydride d'acide maléique, l'anhydride d'acide succinique et l'anhydride d'acide glutarique.

12. Procédé selon l'une des revendications 1 à 11, **caractérisé en ce que** le produit réactionnel est séché.

13. Procédé selon la revendication 12, **caractérisé en ce que** le séchage s'effectue dans une cuve de réaction.

14. Procédé selon la revendication 12 ou 13, **caractérisé en ce que** le séchage s'effectue sous pression réduite et/ou à des températures de 40 à 100°C, de préférence de 60 à 80°C.

15. Utilisation d'un sel préparé selon l'une des revendications 1 à 14 pour la préparation de réactifs photographiques formulés sous forme solide.

16. Utilisation selon la revendication 15, **caractérisée en ce que** le réactif photographique est un bain de blanchiment.

17. Utilisation selon la revendication 15 ou 16, **caractérisée en ce que** les réactifs photographiques sont formulés par granulation fusion.
